# EUROPEAN PATENT APPLICATION

(11) **EP 4 501 231 A1**
(43) Date of publication of application: **05.02.2025**
(21) Application number: 23774230.9
(22) Date of filing: 27.01.2023
(51) Int. Cl.: A61B 5/27, B32B 5/00, B32B 27/18, B32B 27/40, D06M 11/74, D06M 15/564, H01B 5/14

(54) **BIOELECTRODE AND METHOD FOR MANUFACTURING SAME**

(30) Priority: 24.03.2022 JP 2022048043
(71) Applicant: Toray Industries, Inc., Tokyo 103-8666 (JP)
(72) Inventor: TONOMORI, Keiichi, Otsu-shi, Shiga 520-2141 (JP); KAMEMOTO, Chigusa, Otsu-shi, Shiga 520-2141 (JP)
(74) Representative: Mewburn Ellis LLP
(86) International application number: PCT/JP2023/002616
(87) International publication number: WO 2023/181631

(57) **Abstract**

An object is to provide a bioelectrode having a textile form, and which is flexible, highly versatile, excellent in fastness, and curbs lowering in conductivity due to washing, and a method for manufacturing the same, and the gist is to provide a bioelectrode having a multilayer structure of a conductor and a fiber base material composed of non-conductive fibers, in which the conductor contains carbon black and a polyurethane resin, a content of the carbon black is 15 to 35 mass% with respect to the conductor, the carbon black is dispersed in a particulate form at least on a surface of the conductor, a ratio of a longest distance to a shortest distance between particles of the carbon black and adjacent particles of the carbon black (longest distance/shortest distance) is 1 to 20 on the surface of the conductor, and the surface of the conductor has a wet rubbing fastness of grade 4 or higher and a method for manufacturing the same.

## Description

### TECHNICAL FIELD

The present invention relates to a bioelectrode and a method for manufacturing the same.

### BACKGROUND ART

Various types of bioelectrodes, such as gel electrodes, rubber electrodes, electrodes using thin metal plates, and electrodes using conductive fiber materials have been used as bioelectrodes used for measuring bioelectric signals, such as brain waves, electrocardiograms, and electromyograms of human bodies or animals, or for applying electric stimulation to living bodies.

While the above gel electrodes and rubber electrodes have the advantages of being highly flexible, adherable to a body surface of a living body, and able to stably acquire biosignals, they have a problem in that they have poor breathability, which causes a place of contact to become overheated and develop a rash or the like.

In addition, since the electrode using a thin metal plate is hydrophobic and stiff, it has a problem in that it has little suitability for applications in which the electrode comes into contact with a body surface of a living body, which is moisture-rich and flexible. In order to adhere the electrode to the body surface, a high contact pressure is necessary, or it is necessary to use a conductive paste (jelly).

A textile-shaped electrode having conductivity is considered to be effective as an electrode that can be directly attached to a body surface of a living body without using a conductive paste or the like, and various proposals have been made for such an electrode. The textile electrode is resistant to bending and can change its shape in accordance with the unevenness of the body surface.

A conductive polymer coating, the use of a metal fiber, and the use of a carbon material have been proposed in order to impart conductivity in a textile-shaped electrode. As an electrode using a conductive polymer, an electrode in which a polyester fiber base material is impregnated with a conductive polymer (PEDOT-PSS) and an olefin binder has been proposed (Patent Document 1). As an electrode using a metal fiber or a carbon material, an electrode in which carbon black and silicon rubber are arranged on a base material using a stainless steel-containing woven yarn (Patent Document 2), an electrode in which carbon black is impregnated into a base material (Patent Document 3), and an electrode in which a conductive ink containing carbon black is printed on a base material (Patent Document 4) have been proposed.

### PRIOR ART DOCUMENTS

### PATENT DOCUMENTS

Patent Document 1: International Publication No. WO 2017/183463
Patent Document 2: PCT Japanese Translation Patent Publication No. 2006-512128
Patent Document 3: Japanese Patent Laid-open Publication No. 2020-180406
Patent Document 4: PCT Japanese Translation Patent Publication No. 2017-512542

### SUMMARY OF THE INVENTION

### PROBLEMS TO BE SOLVED BY THE INVENTION

The bioelectrode described in Patent Document 1 is formed by impregnating a polyester fiber base material having a single yarn fineness (500 nm or less) with PEDOT-PSS and an olefin binder. Conductivity of an electrode cross section increases by supporting the conductive polymer and the binder between single fibers having a small fineness. Since the olefin material contained in the bioelectrode is excellent in releasability but has weak adhesive strength with respect to the fiber base material, there is room for improvement in washing durability. In addition, PEDOT-PSS used as a conductive polymer is expensive and has a problem in versatility. The bioelectrode described in Patent Document 2 has a base material composed of a conductive yarn such as stainless steel or the like or a base material composed of a conductive wire. In addition, the use of carbon black and silicon rubber (silicone rubber) is exemplified for these bioelectrodes. The base material composed of a conductive yarn such as stainless steel or the like and the base material composed of a conductive wire tend to have poor flexibility compared with a base material composed of non-conductive fibers. In addition, the silicone material included in these bioelectrodes has interfacial characteristics of having excellent releasability. Therefore, these bioelectrodes are likely to separate from the skin during use and the bioelectrodes may move out of position due to the movement of a wearer when acquiring biosignals, and there is a concern that artifacts may be generated in the bioelectrodes that acquire or input weak signals. On the other hand, the bioelectrode described in Patent Document 3 has a configuration in which inexpensive carbon black is impregnated into a fiber base material together with a urethane binder. Since the fiber base material is impregnated with the conductive resin, there is a problem in the stability of the conductivity of an electrode surface in contact with the skin, and there is room for improvement such as with regard to the large difference in impedance between the electrodes before and after washing. The bioelectrode described in Patent Document 4 has a configuration in which a conductive ink containing carbon black and a urethane binder is printed on an elastic material. Since a flexible conductive ink is used, the flexibility is sufficient, but since the amount of conductive particles in the conductive material of these bioelectrodes is large, the rubbing fastness is poor and color migration tends to occur.

In view of the above, an object of the present invention is to provide a bioelectrode having a textile form, and which is flexible, highly versatile, excellent in fastness, and curbs lowering in conductivity due to washing, and a method for manufacturing the same.

### SOLUTIONS TO THE PROBLEMS

The configuration of the invention for solving the above problem and achieving the object is any one of the following.
(1) A bioelectrode having a multilayer structure of a conductor and a fiber base material composed of non-conductive fibers,
   in which the conductor contains carbon black and a polyurethane resin,
   a content of the carbon black is 15 to 35 mass% with respect to the conductor,
   the carbon black is dispersed in a particulate form at least on a surface of the conductor,
   a ratio of a longest distance to a shortest distance between particles of the carbon black and adjacent particles of the carbon black (longest distance/shortest distance) is 1 to 20 on the surface of the conductor, and
   the surface of the conductor has a wet rubbing fastness of grade 4 or higher.
(2) The bioelectrode according to (1), in which a ratio of an area having a spreading resistance value of 4 to 6 logΩ measured by SSRM in the surface of the conductor is 8% to 18%.
(3) The bioelectrode according to (1) or (2), in which a difference in impedance between the electrodes before and after 20 times of washing according to JIS L 0217 (1995) 103 method is 0.2 kΩ or less.
(4) A method for manufacturing a bioelectrode, by which the bioelectrode according to any one of (1) to (3) is manufactured, the method including:
   a step of mixing and defoaming a solution containing carbon black and a polyurethane resin which have been stirred by a homomixer with a planetary ball mill to obtain a coating liquid; and
   a step of applying the coating liquid onto one surface of a fiber base material composed of non-conductive multifilament fibers and drying the coating liquid.

### EFFECTS OF THE INVENTION

According to the present invention, a bioelectrode having a textile form, and which is flexible, highly versatile, excellent in fastness, and curbs lowering in conductivity due to washing is obtained.

### EMBODIMENTS OF THE INVENTION

Hereinafter, an embodiment of a bioelectrode according to the present invention will be described in detail. Note that the present invention is not limited to the embodiment.

### <Bioelectrode>

The bioelectrode of the present invention has a multilayer structure of a conductor and a fiber base material composed of non-conductive fibers, in which the conductor serves as a contact surface with a living body to acquire and transmit biosignals. Here, the above multilayer structure may be a multilayer structure formed by directly laminating the fiber base material and the conductor, or it may be a multilayer structure of 3 or more layers with a mixed layer in which fibers are mixed with a conductor or the like provided between the fiber base material and the conductor.

### <Conductor>

In the present invention, the conductor is a material exhibiting conductivity. The conductor as used herein is a mixture for imparting conductivity to the bioelectrode including (A) carbon black and (B) a polyurethane resin. Also, the mixture and the conductor may include other components in addition to (A) the carbon black and (B) the polyurethane resin. Examples of the other components include, but are not limited to, (C) a water-based thickener, (D) a conductivity improver, (E) a flexibility imparting agent, (F) a surfactant and/or a leveling agent, a crosslinker, a catalyst, an antifoaming agent, or the like. The conductivity as used herein is a surface resistance value of less than 1 × 10¹⁰ Ω in a case where the surface resistance of the bioelectrode is measured by a measurement method described later.

### <(A) Carbon Black>

The carbon black (hereinafter, may be referred to as CB) used in the present invention is a conductive substance. When imparting conductivity by CB, it is crucial to consider a diameter of particles forming a CB base (a particle diameter of particles constituting the structure; hereinafter, the particles may be referred to as primary particles and the particle diameter may be referred to as a primary particle diameter), a structure, which is a chain structure of the primary particles, and particle surface properties. In the case of preparing a solution including CB and a binder, it is particularly important to increase the specific surface area by modifying the particle surface properties. A large specific surface area means that there are many pores on a particle surface, and since the binder enters the pores, a distance between the particles (which will be described later) of CB dispersed in a particulate form in the conductor can be reduced, which leads to achieving high conductivity.

The specific surface area can be measured by a BET method, and the BET specific surface area of the carbon black is preferably 400 to 2000 m²/g or more. The BET specific surface area of the carbon black is more preferably 600 to 1600 m²/g. When the BET specific surface area of the carbon black is 400 m²/g or more, the distance between the particles becomes sufficiently small and is in a range in which sufficient conductivity can be imparted to the bioelectrode, and when it is 600 m²/g or more, an even higher level of conductive performance can be achieved. When an upper limit is 2000 m²/g or less, the structure can be maintained and stable conductive performance can be imparted to the bioelectrode. This effect becomes more significant when the BET specific surface area of the carbon black is 1600 m²/g or less. Examples of commercially available carbon black products having the above BET specific surface area include LIONPASTEs W-310A, W-311N, W-376R, and W-370C.

In addition, in order to achieve high conductivity, it is desirable that the primary particles of CB be small. An average particle diameter of the primary particles of CB is preferably in the range of 1 to 200 nm, and more preferably in the range of 5 to 100 nm. When the average particle diameter of the primary particles of CB is 1 nm or more, robustness of the bioelectrode against friction becomes apparent, and when the average particle diameter is 5 nm or more, a high level of robustness is achieved. When the average particle diameter of the primary particles of CB is 200 nm or less, sufficient conductive performance is imparted to the bioelectrode, and further, when the average particle diameter of the primary particles of CB is 100 nm or less, high conductivity is imparted. The primary particle diameter is obtained by measuring diameters of circles circumscribing a plurality of primary particles arbitrarily selected from an image obtained by observing an ultrathin slice cut off from a conductor by a transmission electron microscope (TEM) at an arbitrary magnification at which the primary particle diameter can be observed, and then averaging those measured values.

Examples of commercially available carbon black products having the above average particle diameter include LIONPASTEs W-310A, W-311N, W-376R, and W-370C.

In the bioelectrode of the present invention, the content of (A) the carbon black is required to be 15 mass% to 35 mass% with respect to 100 mass% of the solid content of the conductor. The content of (A) the carbon black is preferably 20 mass% to 30 mass% with respect to 100 mass% of the solid content of the conductor. As the content of carbon black increases, the higher the conductivity obtained, but as the content increases, the fastness decreases due to friction, leading to not only contamination but also deterioration of performance in actual use. When the content is 15 mass% or more, the conductivity in the bioelectrode is obtained even after washing due to the continuity of the carbon black, and when the content is 20 mass% or more, the conductive performance is further improved. In addition, when the content of CB is 35 mass% or less, or further, 30 mass% or less, robustness of the conductor against friction can be achieved. The solid content as used herein refers to components used for forming the conductor excluding the solvent.

In addition, the carbon black is usually dispersed in a particulate form in the conductor. In order to obtain high conductivity, it is necessary that the carbon black be dispersed in a particulate form at least on the surface of the conductor, and when inter-particle distances between particles (I) of the CB on the conductor surface and adjacent particles of the CB are evaluated, it is necessary that a ratio of a longest distance to a shortest distance (longest distance/shortest distance) be 1 to 20. The ratio of the longest distance to the shortest distance is preferably 1 to 18. Regarding being dispersed in a particulate form described herein, carbon black may be dispersed in units of structures, or may be dispersed in the form of secondary particles in which structures are aggregated. This dispersed structure or mass of secondary particles is treated as particles for evaluating the shortest distance and the longest distance. Regarding the shortest distance and the longest distance, in 360 degrees around a particle of random CB on the conductor surface, a closest distance to an adjacent particle of the CB is the shortest distance, and the farthest distance is the longest distance. A small ratio (longest distance/shortest distance) means that a difference between the shortest distance and the longest distance is small, and means that CB is uniformly dispersed. It goes without saying that the ratio becomes 1 in a case where the shortest distance and the longest distance are equal, and it becomes more preferable as the ratio approaches 1. When setting the ratio of the longest distance to the shortest distance (longest distance/shortest distance) to 20 or less, carbon black is uniformly dispersed on the conductor surface and a conductive path can be efficiently formed, and thus the conductivity increases, whereby the amount of CB used can be reduced, and both rubbing fastness and conductivity can be achieved. Furthermore, by setting the ratio to 18 or less, continuity of carbon black can be maintained even after washing, and the conductivity for a bioelectrode can be obtained. Setting the ratio of the longest distance to the shortest distance within the above range can be achieved by a manufacturing method described later. In addition, it is preferable that the shortest distance between the CB particles be in a range of 10 to 25 nm and the longest distance thereof be in a range of 10 to 500 nm in order to obtain favorable conductivity.

### <(B) Polyurethane Resin>

The conductor further includes (B) a polyurethane resin in addition to (A) the carbon black above. Here, (B) the polyurethane resin plays a role of a binder for supporting the mixture constituting the conductor on the fiber base material. Since the binder significantly contributes to the flexibility of the bioelectrode, it is important that the binder is polyurethane resin. Since polyurethane resin has excellent flexibility compared with resins other than polyurethane resin, the bioelectrode of the present invention is flexible and has excellent comfort when wearing.

(B) The polyurethane resin can prevent the mixture constituting the conductor in the bioelectrode from falling off from the fiber base material, and further, reduce separation of the bioelectrode from the skin and improve biosignal acquisition. From this perspective, one of the characteristics of the present invention is that the conductor includes polyurethane resin.

The conductor may include, as a binder, a polyurethane resin alone or 1 or a plurality of other resins, and preferable specific examples of the other resins include olefin-based resin, polyester-based resin, polyurethane resin, epoxy resin, silicone resin, vinyl chloride resin, nylon resin, acrylic-based resin, and the like.

Polyurethane resins are classified into ether-based, ester-based, carbonate-based, modified polyol, ester/carbonate-based, or a polymer or the like obtained by combining them depending on a raw material polyol, and any of these can be used. From the viewpoint of hydrolysis resistance, it is preferable to include at least one selected from a group consisting of ether-based polyurethane resin and carbonate-based polyurethane resin in particular.

Examples of commercially available products that can be used as the polyurethane resin include the following. That is, examples of ether-based polyurethane resins include "RESAMINE (registered trademark) D-2040" (Dainichiseika) and "SUPERFLEX (registered trademark) E-4800" (DKS), and carbonate-based polyurethane resins include "EVAPHANOL (registered trademark) HA-107C" (NICCA CHEMICAL), "RESAMINE (registered trademark) D-6300" (Dainichiseika), "RESAMINE (registered trademark) D-6065NP" (Dainichiseika), and "SUPERFLEX (registered trademark) 460" (DKS), and further, polyether/carbonate-based polyurethane resins include "RESAMINE (registered trademark) D-4080" (Dainichiseika) and "RESAMINE (registered trademark) D-4200" (Dainichiseika), and the like.

Polyurethane resins include polyurethane resins derived from aromatic isocyanates and aliphatic isocyanates, and from the viewpoint of toxicity and yellowing, polyurethane resins having aliphatic isocyanates are desirable for use in bioelectrodes that contact the skin and require durability. Furthermore, among polyurethane resins having aliphatic isocyanates, aliphatic isocyanate carbonate-based urethanes are more preferable because they have high hydrolysis resistance and flexibility.

In the bioelectrode of the present invention, from the viewpoint of flexibility of the obtained bioelectrode, a glass transition temperature (Tg) of the polyurethane resin is preferably -60 to ±0°C. The glass transition temperature (Tg) is measured based on the following method. First, a resin solution is poured into a stainless steel box and dried at 60°C for 1 hour, then at 120°C for 2 hours to create a film with a thickness of about 0.3 mm. Using this film, a dynamic viscoelasticity of -100 to 200°C is measured with a viscoelasticity measuring apparatus DMS6100 (manufactured by Seiko Instruments Inc.), and a peak temperature (T°C) of an obtained loss viscoelastic modulus is defined as the glass transition temperature (Tg). Note that measurement is carried out at a temperature rising rate of 5°C/min and a measurement frequency of 1 Hz.

In addition, from the viewpoint of friction resistance of the obtained bioelectrode, a tensile strength of the polyurethane resin alone is preferably 5 to 50 MPa. When the tensile strength of the polyurethane resin alone is 5 MPa or more, robustness of the bioelectrode against friction is obtained, and when the tensile strength of the polyurethane resin alone is 50 MPa or less, a flexibility unique to the textile of the bioelectrode can be more reliably maintained.

In the bioelectrode of the present invention, a content of the polyurethane resin is not particularly limited, but is preferably 65 to 85 mass%, and more preferably 70 to 80 mass% per 100 mass% of the solid content of the conductor. When the content of the polyurethane resin is 65 mass% or more, (A) the carbon black included in the bioelectrode is less likely to fall off. On the other hand, when the content of the polyurethane resin is 85 mass% or more, the distance between carbon blacks in the bioelectrode becomes long, and stable conductivity cannot be secured.

### <Fiber Base Material>

As the fibers constituting the fiber base material provided in the bioelectrode of the present invention, non-conductive fibers are used instead of using metal fibers such as stainless steel, aluminum, aluminum alloy, or copper; metal-coated fibers coated with a metal such as silver; conductive fibers spun using a resin kneaded with metal or carbon; or the like. The term "non-conductive" as used herein is a surface resistance value of equal to or more than 1 × 10¹⁰ Ω in a case where the surface resistance of the fiber base material is measured by a method described in Examples described later. Non-conductive fibers are easily available compared with conductive fibers, thus the bioelectrode of the present invention is excellent in terms of productivity.

The non-conductive fibers constituting the fiber base material used in the present invention may be natural fibers or chemical fibers. Examples of natural fibers include cellulose-based fibers such as cotton and hemp, protein fibers such as wool and silk, and the like. Examples of chemical fibers include regenerated fibers such as rayon, semisynthetic fibers such as acetate, synthetic fibers, and the like. From the viewpoint of workability, the fibers constituting the fiber base material used in the present invention are preferably synthetic fibers.

Examples of the synthetic fibers include polyamide fibers such as nylon and aramid, polyester fibers such as polyethylene terephthalate, acrylic fibers such as polyacrylonitrile, polyolefin fibers such as polyethylene and polypropylene, polyvinyl alcohol fibers, polyvinyl chloride-based fibers, polyurethane fibers, polyimide fibers, and other heterocyclic polymer fibers. From the viewpoint that higher flexibility can be imparted to the bioelectrode, it is preferable to use any one or a plurality of types of fibers among polyamide fibers, polyester fibers, and polyolefin fibers in particular.

In the bioelectrode of the present invention, a cross-sectional shape of the fibers constituting the fiber base material may be a round cross-section, a triangular cross-section, a flat cross-section, a polygonal cross-section, a hollow shape, or another irregular cross-sectional shape having a high degree of irregularity, and it is not particularly limited.

Examples of a form of the fiber base material according to the present invention include mesh, paper, woven fabric, knitted fabric, non-woven fabric, ribbon, string, and the like. The form is not particularly limited as long as it is suitable for the purpose of use, but from the viewpoint of conductivity and workability, a woven fabric is desirable.

In the case of a woven fabric, it is desirable that a total of each cover factor of a warp yarn and a weft yarn of a woven fabric, in respect to a cover factor calculated by multiplying a square root of a fineness by a yarn density, be 1500 or more and 3000 or less. When the cover factor is small, not only a coating agent reaches a back surface and there is a possibility that the fiber base material will not function as an insulating layer but also the coating liquid reaching the back surface adheres to production equipment and the coating amount is not stable. On the other hand, in woven fabric having the cover factor of more than 3000, not only a weaving performance deteriorates, but also rigidity increases and flexibility deteriorates.

A form of the fibers may be any of monofilament yarns, multifilament yarns, or staple yarns.

A single fiber fineness is not particularly limited and may be, for example, about 0.0001 dtex to 300 dtex.

The fiber base material is subjected to many treatments such as a shrinkage treatment, a form fixing treatment, a compression treatment, a dyeing finishing treatment, an oil application treatment, a heat fixing treatment, solvent removal, form fixing agent removal, a combing treatment, a polishing treatment, a plane (roll) press treatment, and a high-performance short-cut shearing treatment (cutting raised fibers), in addition to a fiber entangling treatment and a raising treatment, with the treatments appropriately combined in each step, but the implementation is not limited as long as the performance as an electrode is not impaired.

In addition, it is preferable that the fiber base material have a surface resistance value of 1 × 10¹⁰ Ω or more when measuring with the fiber base material alone by a later-described method. When a conductive yarn such as a stainless steel or the like or a conductive wire is used to lower a resistance value of the fiber base material alone and the surface resistance value is set to 1 × 10¹⁰ Ω or less, rigidity is generated in an electrode when a conductor is applied, and there is a possibility that wearing comfort will be impaired. Also, when the fiber base material itself has conductivity, the fiber base material cannot be utilized as an insulating layer. Examples of the fiber base material having a surface resistance value of 1 × 10¹⁸ Ω or more include a fiber base material using a fluorine-based polymer, and the surface resistance value of the fiber base material is preferably 1 × 10¹⁸ Ω or less because there is a concern that the coatability of a processing agent may deteriorate. Note that the surface resistance value of the fiber base material alone can be controlled by the selection of the polymer forming the fiber base material or by limiting the application of the conductive substance during or after forming the base material.

### <Wet Rubbing fastness of Conductor Surface>

A wet rubbing fastness of the conductor surface of the bioelectrode of the present invention needs to be grade 4 or higher. When the fastness is grade 4 or higher, carbon black can be prevented from falling off due to friction during wearing or washing. Such wet rubbing fastness can be achieved by controlling the content of carbon black in the conductor within the above range and uniformly dispersing the carbon black in the binder.

### <Area ratio of Spreading Resistance Value of Conductor Surface>

An area ratio of a high conductive region having a spreading resistance value of 4 to 6 logΩ as measured by scanning spreading resistance microscopy (hereinafter referred to as SSRM) on the conductor surface of the bioelectrode of the present invention is preferably 8% to 18%. When setting the area ratio to 8% or more, it is possible to sufficiently stably acquire an electric signal from a living body, and it is not necessary to take measures to impair comfort such as increasing the pressure. When the area ratio is 18% or less, carbon black as an outermost conductive component is uniformly dispersed, excellent wet rubbing fastness is obtained, and a change in electrical characteristics before and after washing is also reduced. The area ratio can be controlled by using a predetermined amount of carbon and uniformly dispersing the carbon.

### <Impedance between Electrodes>

In the bioelectrode of the present invention, a difference in impedance between the electrodes before and after 20 times of washing according to JIS L 0217 (1995) 103 method is preferably 0.2 kΩ or less. A resistance value or resistivity depends on a residual amount of the conductive component in the entire conductor, but the impedance between the electrodes depends on the residual amount of the conductive component in the outermost layer of the conductor, so that the influence of falling off due to washing becomes more significant. In a case of acquiring more detailed biological information such as medical bioelectrodes, it is important not only to keep the impedance between the electrodes at a low value, but also to reduce a change in the impedance between the electrodes before and after washing. When the difference in impedance between the electrodes between before and after 20 times of washing is 0.2 kΩ or less, accuracy in repeated wearing can be maintained. Furthermore, when setting the difference in impedance between the electrodes before and after 50 times of washing according to JIS L 1930 (2014) C4G method to 0.2 kΩ or less, the accuracy in repeated wearing can be further maintained. The difference in impedance between the electrodes can be controlled by using a predetermined amount of carbon and uniformly dispersing the carbon.

### <Other Mixtures>

As described above, in the bioelectrode of the present invention, the conductor may contain, in addition to (A) the carbon black and (B) the polyurethane resin, as other components, (C) the water-based thickener, (D) the conductivity improver, (E) the softness imparting agent, (F) the surfactant and/or the leveling agent, the crosslinking agent, the catalyst, the antifoaming agent, and the like.

Among them, (C) the water-based thickener can be used to control the coating thickness, the degree of penetration of the coating liquid into the fiber base material, and the coating thickness when a coating liquid for forming a conductor is applied to the fiber base material. When imparting viscosity characteristics to a solution (coating liquid) formed of the formulation constituting the conductor, the thickness of the conductor can be controlled, and a bioelectrode having more excellent conductivity can be obtained.

From the viewpoint of reducing VOC during production, the water-based thickener is usually used by being dissolved in water. One type of water-based thickener may be used, or two or more types may be used in combination.

Examples of the water-based thickener include the following. Inorganic-based examples include silicates and montmorillonites, and organic-based examples include cellulose-based carboxymethyl cellulose, methyl cellulose, hydroxyethyl cellulose, vinyl-based polyvinyl alcohol, polyvinyl pyrrolidone, polyvinyl benzyl ether copolymer, polyacrylic acid based polyacrylic acid or polyacrylate, a poly(meth)acrylic acid-(meth)acrylic acid ester copolymer, a crosslinked core carboxylic acid emulsion, a polyurethane-based polyether-modified urethane compound, a hydrophobically modified polyoxyethylene polyurethane copolymer, a urea-based urethane-urea-based compound, and the like.

Among them, polyacrylic acid based compounds are preferably used because they can impart thixotropic properties to the coating liquid in addition to thickening properties. The coating liquid imparted with thickening and thixotropic properties by the polyacrylic acid based compound provides excellent characteristics, such as preventing sedimentation during storage and improving workability due to a reduction in viscosity during coating.

In the bioelectrode of the present invention, a content of the water-based thickener is not particularly limited, but is preferably 0.1 to 10 mass%, and more preferably 0.5 to 5 mass% per 100 mass% of the solid content of the conductor. When the content of the water-based thickener is 0.1 mass% or more, it is possible to impart thickening properties and thixotropic properties to the coating liquid that forms the conductor of the bioelectrode to improve coatability, and when the content is 10 mass% or less, coating defects due to excessive thickening can be suppressed, so this is preferable.

### <Method for Manufacturing Bioelectrode>

The bioelectrode of the present invention can be produced, for example, by the following procedure. First, a fiber base material composed of non-conductive fibers is produced using non-conductive multifilament fibers or the like. A bioelectrode having a multilayer structure of a conductor and a fiber base material composed of non-conductive fibers can be produced by subjecting to a step of mixing and defoaming a solution containing carbon black dispersion and a polyurethane resin which have been stirred by a homomixer with a planetary ball mill to obtain a coating liquid, and applying the coating liquid onto one surface of the fiber base material composed of non-conductive multifilament fibers and drying the coating liquid. More preferably, it is preferable to stir the carbon black dispersion with a homomixer, then defoam the carbon black dispersion with a vacuum dryer, and mix and defoam the polyurethane resin with a planetary ball mill from the viewpoint of further improving the dispersibility of the carbon black. When the carbon black has insufficient uniformity as a bioelectrode, stirring and mixing conditions are adjusted to improve dispersibility.

As the coating liquid, a dispersion or a solution containing (A) carbon black and a polyurethane resin is preferably used, and the dispersion or the solution is preferably applied onto the fiber base material. Note that in the present specification, a substance that completely dissolves all the components included in the conductor (i.e., a "solvent") and a substance that disperses the insoluble components (i.e., a "dispersion medium") are both referred to as a "solvent" without any particular distinction. The solvent will be described below.

### <Solvent>

The above solvent is not particularly limited and includes, for example, water; alcohols such as methanol, ethanol, 2-propanol, 1-propanol, and glycerin; ethylene glycols such as ethylene glycol, diethylene glycol, triethylene glycol, and tetraethylene glycol; glycol ethers such as ethylene glycol monomethyl ether, ethylene glycol monoethyl ether, ethylene glycol mono-normal butyl ether, diethylene glycol monomethyl ether, ethylene glycol diethyl ether, and diethylene glycol dimethyl ether; glycol ether acetates such as ethylene glycol monoethyl ether acetate, diethylene glycol monoethyl ether acetate, and diethylene glycol monobutyl ether acetate; propylene glycols such as propylene glycol, dipropylene glycol, and tripropylene glycol; propylene glycol ethers such as propylene glycol monomethyl ether, propylene glycol monoethyl ether, dipropylene glycol monomethyl ether, dipropylene glycol monoethyl ether, propylene glycol dimethyl ether, dipropylene glycol dimethyl ether, propylene glycol diethyl ether, and dipropylene glycol diethyl ether; propylene glycol ether acetates such as propylene glycol monomethyl ether acetate, propylene glycol monoethyl ether acetate, dipropylene glycol monomethyl ether acetate, and dipropylene glycol monoethyl ether acetate; tetrahydrofuran; acetone; acetonitrile; and the like. One of these solvents may be used alone, or two or more may be used in combination.

It is preferable that the solvent be water or a mixture of water and an organic solvent. In the case that water is included as the solvent, a content of water is not particularly limited, but is preferably 10 to 1000 mass%, and more preferably 20 to 500 mass% per 100 mass% of the solid content of the conductor. When the content of water is 10 mass% or more, fluidity of the mixture in the solution is ensured and handling is satisfactory, and when the content is 1000 mass% or less, a concentration of the mixture does not drop too low and an amount of coating liquid used does not increase too much.

It is desirable that the carbon black be uniformly dispersed in a solvent in advance. Examples of the method for uniformly dispersing carbon black in a solvent include, for example, a mechanical stirring method using a ball mill, a bead mill, a planetary ball mill, a vibration ball mill, a sand mill, a colloid mill, an attritor, a roll mill, high-speed impeller dispersion, a disperser, a homomixer, a high-speed impact mill, ultrasonic dispersion, a stirring blade, a stirrer, or the like, and it is preferable to perform stirring with a homomixer. Even in a case where a commercially available product in the form of a carbon black dispersion is used, it is more preferable to stir with a homomixer before use in order to obtain a dispersion state suitable for use in the present invention.

Further, it is desirable that dispersibility of carbon black be maintained and bubbles be not contained in a coating liquid obtained by mixing a carbon black dispersion and a polyurethane resin. As a method for mixing and defoaming the carbon black dispersion and the polyurethane resin, it is preferable to perform mixing and defoaming with a planetary ball mill. Carbon black is uniformly dispersed in advance by a homomixer, and then mixed with a polyurethane resin and defoamed by a planetary ball mill to obtain a coating liquid in which carbon black is uniformly dispersed. By using such a coating liquid, a bioelectrode having high conductivity can be obtained even when the amount of carbon black used is reduced.

In the present invention, when the coating liquid is applied onto a fiber base material, a usual method capable of applying the coating liquid onto one surface, such as a pipe coater or a knife coater, may be used. A pipe coater is preferable from the viewpoint of being able to apply the coating liquid with a uniform thickness and obtaining the coating thickness. After the application, it is preferable to volatilize and dry the solvent with heat. After drying, water washing or high temperature treatment may be performed to remove impurities. By applying the coating liquid to a fiber base material and then drying the coating liquid, it is possible to obtain a bioelectrode having a multilayer structure of a conductor and a fiber base material composed of non-conductive fibers.

Since the bioelectrode thus obtained is flexible and has a small change in conductivity due to washing, the bioelectrode is not only excellent in bioelectric signal acquisition performance but also comfortable to wear, and is suitable for measuring bioelectric signals such as electroencephalograms, human electrocardiograms, and electromyograms.

### EXAMPLES

Hereinafter, the bioelectrode of the present invention will be described in detail with reference to the examples. The bioelectrode of the present invention is not limited to these examples. The measured values in the examples and comparative examples are obtained by the following methods.

### <Measurement of Distance between Particles of Carbon Black and Calculation of Ratio between Shortest Distance and Longest Distance>

The distance between the particles of carbon black can be determined by subjecting an ultrathin slice cut out from the conductor surface of the bioelectrode to measurement with a transmission electron microscope (TEM).

The ultrathin slice was cut out so as to have a thickness two times the primary particle diameter of CB. In a case where the primary particle diameter of the CB is unknown, an ultrathin slice is preliminarily cut out with a plurality of thicknesses and subjected to surface observation, a diameter of the largest particle that can be confirmed is defined as a provisional primary particle diameter, the ultrathin slice is cut out with a thickness that is two times, three times, or four times the provisional primary particle diameter and subjected to surface observation, and in a case where the difference between the diameters of the largest particles that can be confirmed in each image is 10 nm or less, the average value of the diameters is used as the primary particle diameter, and the thickness of the ultrathin slice cut out and the visual field to be observed are determined. When the difference between the diameters of individual images is more than 10 nm, the diameter of the largest particle among them is defined as the provisional primary particle diameter, and an operation of cutting an ultrathin slice at a thickness of two times, three times, or four times the particle diameter and observing the surface is repeated.

In an image observed and acquired in a field of view of 40 times the primary particle diameter of the CB using TEM, three random points of particles of CB are selected from a range in which a radius is three times the primary particle diameter around an intersection of diagonal lines of the image. For each selected particle, a distance from an outer edge to an outer edge of the adjacent particle of the CB was measured at a circumference of 360 degrees, and a distance to the closest particle was defined as the shortest distance, and a distance to the farthest particle was defined as the longest distance. For the particles at the three selected points, an average of the obtained shortest distances was taken as the average shortest distance, and an average of the obtained longest distances was taken as the average longest distance. A ratio of the average longest distance to the average shortest distance was defined as the ratio of the shortest distance to the longest distance (longest distance/shortest distance). The "adjacent particle of CB" is a particle of CB in which at least a part of an outer edge thereof is included in the image.

### <Wet Rubbing fastness>

The bioelectrode was subjected to a wet test (a test piece was left in a standard state for 4 hours or longer, and a white cotton fabric for friction was wetted with water by about 100%) according to JIS L 0849 (1996) (here, 100 reciprocating friction in the standard is defined as 30 reciprocating friction) using a friction tester Type II (Gakushin type), and a coloring determination was performed by grade 1 to grade 5 in gray scale determination for staining.

### <Ratio of Area Where Spreading Resistance Value Is 4 to 6 logΩ>

A ratio of area where the spreading resistance value was 4 to 6 logΩ in the conductor surface of the bioelectrode of the present invention was determined as follows. Using a scanning spreading resistance microscope (SSRM), a voltage was applied to the conductor surface of the bioelectrode from the back side of a sample for measurement, and using a conductive probe, the conductivity of a region of 60 µm × 60 µm in a surface layer of the sample was observed. Using image processing software (GIMP 2.8 portable), an area ratio of a portion having a spreading resistance value of 4 to 6 logΩ in the measured region was determined. In this case, as the number to be observed, three randomly selected surfaces were measured. An average value of the area ratios obtained at the three portions was calculated, and this was taken as the "a ratio of an area where the spreading resistance value of the conductor surface is 4 to 6 logΩ".
Observation device: manufactured by Digital Instruments by Bruker AXS
   NanoScope Iva AFM
   Dimension 3100 Stage AFM System + SSRM Options
SSRM scan mode: Simultaneous measurement of contact mode and spreading resistance
SSRM probe (Tip): Diamond coated silicon cantilever
Probe part number: DDESP-FM (manufactured by Bruker AXS)
Ar ion beam machining apparatus: IM-4000 acceleration voltage 3 kV manufactured by Hitachi High-Technologies Corporation

### <Washing Method>

Washing treatment was performed by the following two methods.
1. After 20 times of washing according to the JIS L 0217 (1995) 103 method, a fabric was hung and dried.
2. After 50 times of washing according to JIS L 1930 (2014) C4G method, a fabric was hung and dried.

### <Impedance between Electrodes>

Conductors of the bioelectrode were brought into contact with each other, a pressure of 31.3 g per square centimeter was applied, and an impedance between the electrodes (kΩ) was measured according to ANSI 4.2.2.1. The measurement was performed three times, and an average value thereof was taken as the impedance between the electrodes.

### <Surface Resistance Value of Fiber Base Material>

The surface resistance value (Ω) of the fiber base material of the bioelectrode was measured using a surface electrical resistor (Megaresta H0709 manufactured by SHISHIDO ELECTROSTATIC, LTD.) under an environment of 20°C and 40% RH at 500 V for 60 seconds. The value was measured at three points and averaged.

### [Example 1]

A plain woven fabric was woven using polyester filaments (made of polyethylene terephthalate (PET)) of 84 dtex-72 F as warps and wefts. The obtained woven fabric is scoured with an aqueous solution of 0.5 g/L of a surfactant and 0.7 g/L of sodium hydroxide (at 80°C for 20 minutes), washed with water (at 50°C for 10 minutes), and heat-set (at 180°C for 1 minute) using a dry heat treatment machine to obtain the fiber base material having weaving density (180 warp threads/2.54 cm and 94 weft threads/2.54 cm). A carbon black dispersion ""LION PASTE (registered trademark)" W-376R" (manufactured by LION SPECIALTY CHEMICAL CO., LTD., solid content: 12.5%) was stirred with a homomixer for 15 minutes, and then defoamed with a vacuum dryer, and 408 g/L of the carbon black dispersion was mixed with 389 g/L of an aqueous polyurethane resin ""SuperFlex (registered trademark) 460"" (aliphatic isocyanate carbonate-based urethane, solid content: 38%, manufactured by DKS Co. Ltd.), 11 g/L of an water-based thickener "PRINGEN NFV" (manufactured by Matsui Dye Chemical Co., Ltd., solid content: 45%), and ion-exchanged water as a solvent so as to be 1 L in total, and the mixture was stirred and defoamed for 90 seconds with a planetary ball mill to obtain a coating liquid. The obtained coating liquid was applied to a fiber base material with a pipe coater, heated at 130°C, then washed with water, and set at 170°C to obtain a bioelectrode having a conductor adhesion amount of 70 g/m². The properties of used parts and the obtained bioelectrode are illustrated in Table 1.

### [Example 2]

A bioelectrode having a conductor adhesion amount of 70 g/m² was manufactured by performing the same treatment as in Example 1 except that the use amount was changed to 326 g/L of the carbon black dispersion and 416 g/L of the aqueous polyurethane resin. The properties of used parts and the obtained bioelectrode are illustrated in Table 1.

### [Example 3]

A bioelectrode having a conductor adhesion amount of 70 g/m² was manufactured by performing the same treatment as in Example 1 except that the use amount was changed to 538 g/L of the carbon black dispersion and 346 g/L of the aqueous polyurethane resin. The properties of used parts and the obtained bioelectrode are illustrated in Table 1.

### [Example 4]

A bioelectrode having a conductor adhesion amount of 70 g/m² was manufactured by performing the same treatment as in Example 1 except that the use amount was changed to 277 g/L of the carbon black dispersion and 432 g/L of the aqueous polyurethane resin and the mixing and defoaming time at the planetary ball mill was changed to 45 seconds. The properties of used parts and the obtained bioelectrode are illustrated in Table 1.

### [Example 5]

A bioelectrode having a conductor adhesion amount of 70 g/m² was manufactured by performing the same treatment as in Example 1 except that the use amount was changed to 571 g/L of the carbon black dispersion and 335 g/L of the aqueous polyurethane resin. The properties of used parts and the obtained bioelectrode are illustrated in Table 1.

### [Comparative Example 1]

A bioelectrode having a conductor adhesion amount of 70 g/m² was manufactured by performing the same treatment as in Example 1 except that the use amount was changed to 212 g/L of the carbon black dispersion and 454 g/L of the aqueous polyurethane resin. The properties of used parts and the obtained bioelectrode are illustrated in Table 1.

### [Comparative Example 2]

A bioelectrode having a conductor adhesion amount of 70 g/m² was manufactured by performing the same treatment as in Example 1 except that the use amount was changed to 652 g/L of the carbon black dispersion and 309 g/L of the aqueous polyurethane resin. The properties of used parts and the obtained bioelectrode are illustrated in Table 1.

### [Comparative Example 3]

A bioelectrode having a conductor adhesion amount of 70 g/m² was manufactured by performing the same treatment as in Example 1 except that stirring was performed for 15 minutes using a stirrer without using a homomixer for stirring the carbon black dispersion, and the carbon black dispersion, the aqueous polyurethane resin, and the water-based thickener were mixed for 90 seconds using a stirrer without defoaming. The properties of used parts and the obtained bioelectrode are illustrated in Table 1.

**[Table 1-1]**

| | | | Example 1 | Example 2 | Example 3 | Example 4 | Example 5 |
|---|---|---|---|---|---|---|---|
| Used parts | Fiber base material | | PET woven fabric | PET woven fabric | PET woven fabric | PET woven fabric | PET woven fabric |
| | Conductive component | | CB | CB | CB | CB | CB |
| | Binder component | | PC-based urethane | PC-based urethane | PC-based urethane | PC-based urethane | PC-based urethane |
| | Binder Tg (°C) | | -21 | -21 | -21 | -21 | -21 |
| | Binder tensile strength (Mpa) | | 25 | 25 | 25 | 25 | 25 |
| Surface resistance value of fiber base material (Ω) | | | > 9.9 × 10¹² | > 9.9 × 10¹² | > 9.9 × 10¹² | > 9.9 × 10¹² | > 9.9 × 10¹² |
| Ratio of conductive component in conductor (mass%) | | | 25 | 20 | 33 | 17 | 35 |
| Ratio of inter-particle distances of carbon black | Average shortest distance (nm) | | 22 | 24 | 18 | 23 | 15 |
| | Average longest distance (nm) | | 308 | 427 | 162 | 437 | 120 |
| | Ratio (longest distance/shortest distance) | | 14 | 18 | 9 | 19 | 8 |
| Wet friction fastness | | | 4 - 5 | 4 - 5 | 4 | 4 - 5 | 4 |
| Ratio (%) of area having spreading resistance value of 4 to 6 logΩ | | | 14.2 | 10.4 | 16.2 | 7.32 | 19.1 |
| Impedance (kΩ) | Before washing | | 0.2 | 0.2 | 0.2 | 0.4 | 0.2 |
| | After 20 times of washing (JIS L 0217) | | 0.3 | 0.2 | 0.4 | 0.5 | 0.4 |
| | | Difference between before and after 20 times of washing | 0.1 | 0 | 0.2 | 0.1 | 0.2 |
| | After 50 times of washing (JIS L 1930) | | 0.3 | 0.2 | 0.6 | 0.7 | 0.7 |
| | | Difference between before and after 50 times of washing | 0.1 | 0 | 0.4 | 0.3 | 0.5 |
| Method for dispersing carbon black | Homomixer | | Provided | Provided | Provided | Provided | Provided |
| | Stirrer | | Not provided | Not provided | Not provided | Not provided | Not provided |
| | Defoaming | | Provided | Provided | Provided | Provided | Provided |
| Method for mixing with binder | Planetary ball mill | | Provided | Provided | Provided | Provided | Provided |
| | Stirrer | | Not provided | Not provided | Not provided | Not provided | Not provided |

**[Table 1-2]**

| | | | Comparative Example 1 | Comparative Example 2 | Comparative Example 3 |
|---|---|---|---|---|---|
| Used parts | Fiber base material | | PET woven fabric | PET woven fabric | PET woven fabric |
| | Conductive component | | CB | CB | CB |
| | Binder component | | PC-based urethane | PC-based urethane | PC-based urethane |
| | Binder Tg (°C) | | -21 | -21 | -21 |
| | Binder tensile strength (Mpa) | | 25 | 25 | 25 |
| Surface resistance value of fiber base material (Ω) | | | > 9.9 × 10¹² | > 9.9 × 10¹² | > 9.9 × 10¹² |
| Ratio of conductive component in conductor (mass%) | | | 13 | 40 | 25 |
| Ratio of inter-particle distances of carbon black | Average shortest distance (nm) | | 26 | 13 | 23 |
| | Average longest distance (nm) | | 624 | 78 | 498 |
| | Ratio (longest distance/shortest distance) | | 24 | 6 | 22 |
| Wet friction fastness | | | 4 - 5 | 3 | 3 |
| Ratio (%) of area having spreading resistance value of 4 to 6 logΩ | | | 7.10 | 20.4 | 2.62 |
| Impedance (kΩ) | Before washing | | 0.6 | 0.2 | 0.4 |
| | After 20 times of washing (JIS L 0217) | | 0.6 | 0.5 | 0.9 |
| | | Difference between before and after 20 times of washing | 0 | 0.3 | 0.5 |
| | After 50 times of washing (JIS L 1930) | | 0.9 | 0.7 | 1.1 |
| | | Difference between before and after 50 times of washing | 0.3 | 0.5 | 0.7 |
| Method for dispersing carbon black | Homomixer | | Provided | Provided | Not provided |
| | Stirrer | | Not provided | Not provided | Provided |
| | Defoaming | | Provided | Provided | Not provided |
| Method for mixing with binder | Planetary ball mill | | Provided | Provided | Not provided |
| | Stirrer | | Not provided | Not provided | Provided |

In the bioelectrode of Examples, carbon black was dispersed in a particulate form on the conductor surface, a ratio of the longest distance to the shortest distance between the carbon black particles and the adjacent particles of carbon black (hereinafter referred to as "ratio between inter-particle distances") was small, a ratio of an area having high conductivity in terms of a spreading resistance value was large, a change in impedance between the electrodes before and after washing was also small, and high biosignal acquisition performance as a bioelectrode was exhibited. In spite of high conductivity, it had excellent wet rubbing fastness. On the other hand, in Comparative Example 1, the amount of carbon black as a conductive component was small, the ratio of the inter-particle distances of carbon black was large, the high conductive region was small, the impedance between the electrodes was high, and the biosignal acquisition performance was poor. In Comparative Example 2, the amount of carbon black as a conductive component was large, and the conductivity was excellent, but the wet rubbing fastness was low, the change in impedance between the electrodes before and after washing was large, and a stable biosignal could not be acquired before and after washing. In Comparative Example 3, since the dispersing of carbon black was insufficient, the ratio of the inter-particle distances of carbon black was large, the high conductive region was small, the change in the impedance between the electrodes before and after washing was also large, the biosignal acquisition performance was poor, and the wet rubbing fastness was also low.

### INDUSTRIAL APPLICABILITY

According to the present invention, it is possible to provide a bioelectrode having a textile form, which is flexible, has high clothing comfort, has a small performance difference before and after washing, and can be repeatedly used. Thus, the bioelectrode of the present invention is suitably used as a bioelectrode for measuring bioelectric signals such as brain waves, electrocardiograms, or electromyograms of human bodies or animals, or for applying electric stimulation to a living body.

## Claims

1. A bioelectrode having a multilayer structure of a conductor and a fiber base material composed of non-conductive fibers,
wherein the conductor contains carbon black and a polyurethane resin,
a content of the carbon black is 15 to 35 mass% with respect to the conductor,
the carbon black is dispersed in a particulate form at least on a surface of the conductor,
a ratio of a longest distance to a shortest distance between particles of the carbon black and adjacent particles of the carbon black (longest distance/shortest distance) is 1 to 20 on the surface of the conductor, and
the surface of the conductor has a wet rubbing fastness of grade 4 or higher.

2. The bioelectrode according to claim **1,** wherein a ratio of an area having a spreading resistance value of 4 to 6 logΩ measured by SSRM in the surface of the conductor is 8% to 18%.

3. The bioelectrode according to claim 1 or **2,** wherein a difference in impedance between the electrodes before and after 20 times of washing according to JIS L 0217 (1995) 103 method is 0.2 kΩ or less.

4. A method for manufacturing a bioelectrode, by which the bioelectrode according to any one of claims 1 to 3 is manufactured, the method comprising:
a step of mixing and defoaming a solution containing carbon black and a polyurethane resin which have been stirred by a homomixer with a planetary ball mill to obtain a coating liquid; and
a step of applying the coating liquid onto one surface of a fiber base material composed of non-conductive multifilament fibers and drying the coating liquid.
